# EUROPEAN PATENT APPLICATION

(11) **EP 1 772 135 A1**
(43) Date of publication of application: **11.04.2007**
(21) Application number: 06076838.9
(22) Date of filing: 05.10.2006
(51) Int. Cl.: A61H 23/02

(54) **Device for transmitting vibrations onto a person or animal and method for its use**

(30) Priority: 06.10.2005 BE 200500492
(71) Applicant: Gymnauniphy, Naamloze Vennootschap, 3740 Bilzen (BE)
(72) Inventor: Temmerman, Frank P. P., 3668 Niel-bij-As (BE); Ex, Johan, 3010 Kessel-Lo (BE); Hostens, Ivo, 3010 Kessel-Lo (BE)
(74) Representative: Donné, Eddy

(57) **Abstract**

Device for transmitting vibrations onto a person or animal, which device (1) mainly consists of a vibrating element (3) and of a drive (8) which makes it possible to transmit a vibration onto the vibrating element (3), characterised in that the device (1) comprises means (9) to determine a measure of the damping which the above-mentioned vibrating element (3) undergoes when in use.

## Description

The present invention concerns a device for transmitting vibrations onto a person or animal.

The present invention concerns a device for training muscles or for stimulating the metabolism of a person or animal, by subjecting the muscular/skeletal system of the person or the animal to vibrations.

Devices for transmitting vibrations onto muscles are already known in different embodiments and mainly consist of a vibration element and of vibration means which make it possible to make this element vibrate at a desired frequency and/or accelerating amplitude.

The purpose of such a device is to make one or several muscles of the human body vibrate, which results in reflex contractions of the muscle or muscles concerned.

By regularly arousing such contractions, the muscle or muscles can be trained, for example in order to make a muscle recover.

Since the intensity of these contractions is a measure for the efficiency of the training, it is known to equip the above-mentioned device with what is called an electromyograph with which the contractions of the muscles can be measured directly.

A disadvantage is that such an electromyograph is relatively expensive.

Moreover, the use of an electromyograph is rather time-consuming, since electrodes must be provided on the muscle or muscles concerned to this end. Moreover, an electromyograph only makes it possible to measure the contractions of the muscles.

The present invention aims to remedy one or several of the above-mentioned and other disadvantages.

To this end, the invention concerns a device for transmitting vibrations onto a person or animal, which device mainly consists of a vibrating element and of a drive which makes it possible to transmit a vibration to the vibrating element, whereby the device comprises means to determine a measure for the damping experienced by the above-mentioned vibrating element when in use.

An advantage of the present invention is that it becomes possible to measure the efficiency of the training of the muscle or muscles in a simple manner. Moreover, it can be easily extended to the entire muscular/skeletal system of a person or animal.

Indeed, intensive research has shown that the aroused muscle contractions dampen the vibration of the vibrating element and that the degree of the damping depends on these contractions and consequently on the efficiency of the training of the muscles.

Moreover, the device makes it possible to determine the frequency or frequencies of the vibrations for a specific user, causing the largest muscle contraction with vibrations having an accelerating amplitude that is as low as possible. Training the muscles with vibrations having an accelerating amplitude that is as low as possible, i.e. with a minimal vibration input, is important in this case since vibrations with a high accelerating amplitude, i.e. vibrations with a large accelerating amplitude or a high frequency may be harmful to the user's health.

Another advantage is that the above-mentioned means can be fixed to the vibrating element and are independent of the person making use of the element, or in other words do not have to be fixed to the person himself. Moreover, the vibrating plate according to the invention makes it possible to measure the intensity of the damping provoked by a person's muscular/skeletal system, whereas measurements with an electromyograph only allow to measure muscle contractions.

From what precedes follows among others that a device according to the invention requires less precautions to guarantee the user the necessary hygiene.

The above-mentioned means for measuring the damping of the vibrating element preferably consist of an accelerometer which is fixed to the vibrating element and of means which make it possible to determine the power of the drive.

An advantage of the use of an accelerometer is that it is relatively cheap compared to an electromyograph.

The present invention also concerns a method for the use of the above-mentioned device.

This method according to the invention for transmitting vibrations onto a person or animal mainly consists in having a user take place at or on the vibrating element and in subjecting the vibrating element to a vibration with a constantly variable frequency and/or accelerating amplitude and in measuring the damping felt by the vibrating element caused by the user, so as to determine an appropriate frequency and/or accelerating amplitude at which a user's muscle or muscles can be optimally trained or at which the user's muscular-skeletal system can be optimally stimulated.

In order to better explain the characteristics of the present invention, the following preferred embodiment of a device according to the invention for transmitting vibrations onto a person or animal, as well as a preferred method for the use of such a device are given as an example only without being limitative in any way, with reference to the accompanying drawings, in which:
figure 1 schematically represents a device according to the invention;
figure 2 is a graphical representation of measuring results which can be obtained when using the device; figure 3 represents a variant of figure 1.

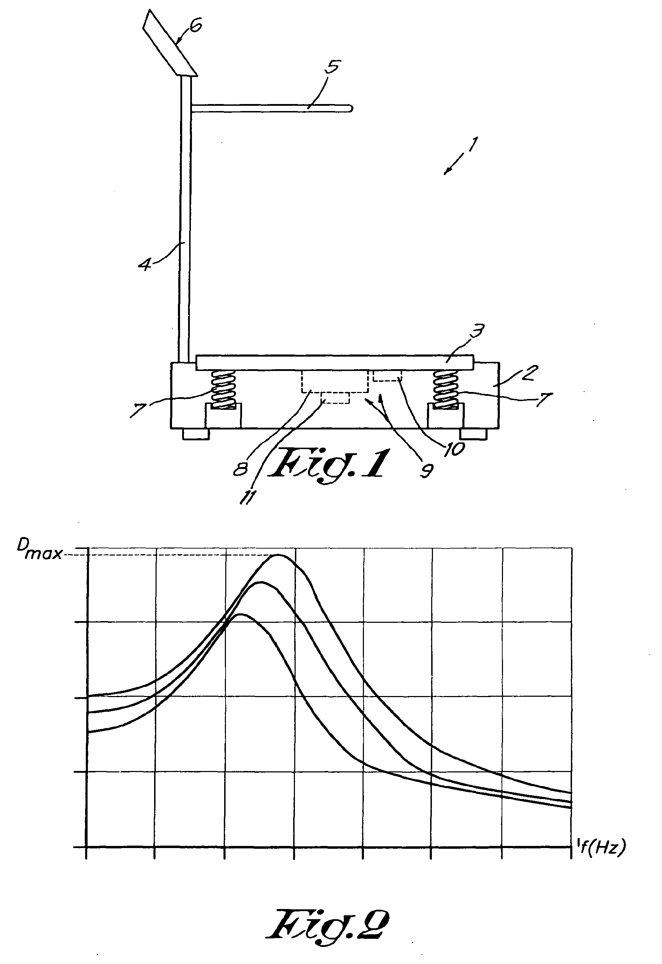

Figure 1 is a device 1 according to the invention which in this case consists of a base 2 onto which a vibrating element 3 is provided in a moving manner.

On the base 2 is in this case provided a standing strut 4 onto which are fixed one or two cross bars 5 and onto which is provided a control panel 6 at the free end.

The above-mentioned vibrating element 3 in this case consists of a rigid plate which is fixed in a moving manner with the base 2 by means of springs 7, for example.

The vibrating element 3 is further provided with a drive 8 which makes it possible to transmit a vibration at a desired frequency and/or accelerating amplitude onto said element 3. Such a drive 8 may for example consist of two imbalance engines rotating in an opposite direction and which can be controlled via the above-mentioned control panel 6.

According to the invention, the vibrating element 3 is provided with means 9 which make it possible to determine the damping that the vibrating element 3 undergoes during the operation.

To this end, the above-mentioned means 9 may consist among others of an accelerometer 10 which is provided on the vibrating element 3 and of a wattmeter 11 or the like, with which the power of the drive 8 can be measured.

The above-mentioned accelerometer 10 and wattmeter 11 are coupled to an arithmetic unit in the control panel 6, which arithmetic unit comprises software which makes it possible to calculate a measure for the damping which the vibrating element 3 undergoes on the basis of the measured quantities.

The working of the above-described device according to the invention is simple and as follows.

In order to use the device 1 according to the invention, the vibrating element 3 must be idly driven at different frequencies and/or accelerating amplitudes, whereby the acceleration of the vibrating element 3 and the power of the drive 8 are measured.

Next, the results of these measurements are stored in a memory, so that one can always check what speed of the vibrating element 3 corresponds to a specific driving power.

Subsequently, the device can be applied to train one or several muscles of a person. To this end, this person must take place on the vibrating element 3 in a specific position, whereby the person can either or not hold on the bars 5.

Then, the drive 8 of the vibrating element 3 is switched on and this drive 8 is controlled such that the frequency of the vibration imposed on the vibrating element 3 is varied over a preset range, for example between 0 and 70 Hertz, preferably at a constant accelerating amplitude.

During this variation of the frequency imposed by the drive 8, both the acceleration of the vibrating plate and the driving power are measured, whereby the measured acceleration is compared to the acceleration of the vibrating element at an identical driving power when the vibrating element is idling. The difference between both accelerations is a measure for the damping which the vibrating element undergoes as a result of the person taking place on or at the vibrating element.

It has been proven by experience that this measure for the damping of the vibrating element varies as a function of the frequency and the accelerating amplitude with which the vibrating element is driven.

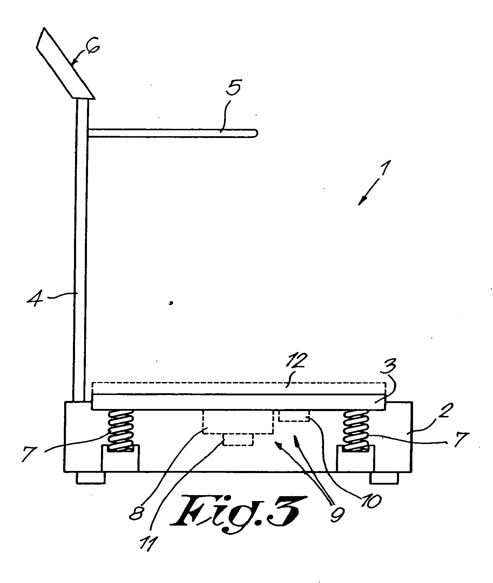

An example of this variation is represented in the graph in figure 2, in which, for different constant accelerating amplitudes, the measure for the damping of the vibrating element 3 is marked off as a function of the imposed frequency.

The above-mentioned graph, which is represented in figure 2, is preferably represented on a screen in the control panel 6.

Intensive research by the applicant has proven that, when driving the vibrating element at the frequency which corresponds to a maximum damping Dₘₐₓ of the vibrating element 3, the largest contractions occur in the muscle or muscles to be trained, which leads us to assume that at the imposed frequency corresponding to said maximum damping Dmₐₓ the muscle or muscles can be trained most efficiently.

This frequency can then also be applied to further train the muscle or muscles concerned.

Naturally, the determination of the most efficient frequency for training the muscles can be repeated at different imposed accelerating amplitudes, as a result of which the most efficient combination of frequency and accelerating amplitude can be determined.

It should be noted that the optimal frequency and accelerating amplitude for training a certain muscle or certain muscles depends on the muscle or muscles concerned; on the person; on the position taken by the person on the vibrating element 3; and on to what extent the muscle or muscles concerned are trained. This leads us to conclude that determining at what frequency and accelerating amplitude a muscle or muscles can be best trained is preferably repeated at the start of every training session.

Next, it should be noted that the above-mentioned means 9 to determine what damping the vibrating element 3 undergoes always consist of a sensor which makes it possible to determine the vibration of the vibrating element 3 and means which make it possible to determine the power of the drive 8 or means which make it possible to determine the force exerted by the person on the vibrating element.

Examples of a sensor for determining the vibration of the vibrating element 3 are for example the above-mentioned accelerometer 10 or a position sensor which can be provided on the vibrating element 3.

The means for determining the power exerted by the drive may consist among others of the above-mentioned wattmeter 11. Means for determining the force exerted by the person on the vibrating element 3 can be realised, as is represented in figure 3, in the form of a dynamometer 12 which makes it possible to measure the force exerted by a user on the vibrating element. Such a dynamometer 12 may for example consist of a balance with piezo-electric elements which are fixed to the vibrating element 3 and which serve as a contact surface for the user.

By making a table prior to the use of the device 1 according to the invention which makes it possible to determine the power exerted by the drive 8 as a function of the measured force, the damping of the vibrating element 3 may again be determined.

Although the devices and method described above by way of example are focussed on the training of muscles, it is clear that such a device and method can also be applied to stimulate the muscular/skeletal system of a person or animal, so as to prevent atrophy or to stimulate the production of certain hormones which may have a positive effect on the metabolism of a human being or animal.

Finally, it should be noted that the vibrating element 3 must not necessarily be made in the form of a plate on which a person can stand, but that the vibrating element 3 may just as well be a handle, a neck support or the like.

The present invention is by no means limited to the above-described embodiment represented in the accompanying drawings; on the contrary, such a device according to the invention for transmitting vibrations onto a person or animal can be realised according to different variants while still remaining within the scope of the invention.

## Claims

1. Device for transmitting vibrations onto a person or animal, which device (1) mainly consists of a vibrating element (3) and of a drive (8) which makes it possible to transmit a vibration onto the vibrating element (3), **characterised in that** the device (1) comprises means (9) to determine a measure of the damping which the above-mentioned vibrating element (3) undergoes when in use.

2. Device according to claim 1, **characterised in that** the above-mentioned means (9) for determining a measure for the damping which the vibrating element (3) undergoes consist of a sensor to determine the vibration of the plate and of means to determine the power exerted by the drive (8) or means for determining the force exerted by a user on the vibrating element (3).

3. Device according to claim 2, **characterised in that** the above-mentioned sensor is an accelerometer (10) or a position sensor.

4. Device according to claim 2, **characterised in that** the above-mentioned means for determining the power exerted by the drive (8) consist of a wattmeter (11) which is connected to the drive (8).

5. Device according to claim 2, **characterised in that** the above-mentioned means for determining the force exerted by a user on the vibrating element (3) comprise a dynamometer (12) provided on the vibrating element (3).

6. Device according to any one of the preceding claims, **characterised in that** it comprises an arithmetic unit with software which makes it possible to calculate the damping of the vibrating element (3) on the basis of the measured quantities.

7. Device according to claim 1, **characterised in that** the vibrating element (3) consists of a rigid plate which is fixed to a base (2) by means of springs.

8. Method for the use of a device according to any one of the preceding claims, **characterised in that** it mainly consists in having a user take place at or on the vibrating element (3) and in subjecting the vibrating element (3) to a vibration at a constantly variable frequency and/or accelerating amplitude and in measuring the damping felt by the vibrating element (3) caused by the user, so as to determine an appropriate frequency and/or accelerating amplitude at which a user's muscle or muscles can be optimally trained or at which the user's muscular-skeletal system can be optimally stimulated.

9. Method according to claim 8, **characterised in that** the muscles of the user are further trained at the frequency and/or accelerating amplitude which corresponds to a maximum damping of the vibrating element (3).

10. Method according to claim 8, **characterised in that** the determination of the imposed frequency which corresponds to a maximum damping (Dₘₐₓ) is repeated at different accelerating amplitudes.

11. Method according to claim 8, **characterised in that** the frequency and/or accelerating amplitude which correspond to a maximum damping {Dmₐₓ) of the vibrating element (3) are determined at the start of a training session for a muscle or muscles.
